# EUROPÄISCHE PATENTSCHRIFT

(11) **EP 1 219 589 B1**
(45) Veröffentlichungstag und Bekanntmachung des Hinweises auf die Patenterteilung: **12.11.2003**
(21) Anmeldenummer: 01129213.3
(22) Anmeldetag: 10.12.2001
(51) Int. Cl.: C07C 68/02, C07C 69/96

(54) **Kontinuierliches Verfahren zur Herstellung von Kohlensäurediarylester**
Continuous process for preparing diaryl esters of carbonic acid [1997/29]
Procédé continu de préparation d'esters diaryliques d'acide carbonique

(30) Priorität: 21.12.2000 DE 10063869
(43) Veröffentlichungstag der Anmeldung: 03.07.2002
(73) Patentinhaber: BAYER AG, 51368 Leverkusen (DE)
(72) Erfinder: Chrisochoou, Andreas, Dr., 51061 Köln (DE); Kühling, Steffen, Dr., 40670 Meerbusch (DE); Vanden Eynde, Johan, 9052 Zwijnaarde (BE)

(56) Entgegenhaltungen:
- EP-A- 0 757 029
- US-A- 4 016 190

## Beschreibung

Gegenstand der vorliegenden Erfindung ist ein neues Verfahren zur Herstellung von Kohlensäurediarylestern, bei denen die Kohlensäurediarylester durch Reaktion von Monophenolen und Phosgen in einem inerten Lösungsmittel in Gegenwart von Alkali und einem Stickstoffkatalysator in der Phasengrenzfläche hergestellt werden.

Die Herstellung von Kohlensäurediarylestern durch den Phasengrenzflächenprozess ist prinzipiell in der Literatur, z.B. Chemistry and Physics of Polycarbonates, Polymer Reviews, H. Schnell, Vol. 9, John Wiley and Sons, Inc. (1964), S. 50/51 vorbeschrieben. Es ist dem Fachmann bekannt und in obengenannter Literatur und beispielsweise in der US 4 016 190 beschrieben, dass eine gute Mischung der beiden Phasen zum Reaktionsfortschritt notwendig ist. Dennoch werden in den bekannten Verfahren nicht immer zufriedenstellende Ergebnisse bezüglich der Ausbeute und Reinheit, insbesondere des Gehalts an Chlorameisensäurearylester, des erhaltenen Kohlensäurediarylesters erzielt.

Ausgehend vom Stand der Technik, insbesondere US 4 016 190, stellte sich die Aufgabe, ein Verfahren bereitzustellen, welches bessere Ausbeuten und Reinheiten an Kohlensäurediarylester ermöglicht. Besonders wichtig ist die Vermeidung der Verschleppung des Chlorameisensäurearylesters ins Endprodukt, da diese Verbindung beim Schmelzeumesterungsprozess zum Polycarbonat stört.

Es wurde nun überraschenderweise gefunden, dass bei der kontinuierlichen Herstellung von Kohlensäurediarylestern durch Reaktion von Monophenolen und Phosgen in einem inerten Lösungsmittel in Gegenwart von Alkali in der Phasengrenzfläche das Einhalten einer bestimmten Mischleistung, die sich innerhalb eines durch untere und obere Grenzwerte definierten Fensters bewegt, notwendig ist, um eine optimale Umsetzung in kurzen Reaktionszeiten mit hohen Ausbeuten und zufriedenstellenden Reinheiten des Kohlensäurediarylesters bei niedrigen Temperaturen zu erzielen.

Hierbei wurde überdies gefunden, dass abhängig von Reaktions-Stufe/-Fortschritt ein jeweils optimaler Mischbereich existiert.

Das eingesetzte Alkali kann eine Alkalilauge (Na, K, Li, Ca-hydroxid) sein, bevorzugt ist Natronlauge und wird im erfindungsgemäßen Verfahren vorzugsweise als 20-55 gew.-%ige, besonders bevorzugt 30-50 gew.-%ige Lösung eingesetzt.

Phosgen kann flüssig, gasförmig oder gelöst im inerten Lösungsmittel eingesetzt werden.

Geeignete Monophenole zum Einsatz in die Reaktion sind 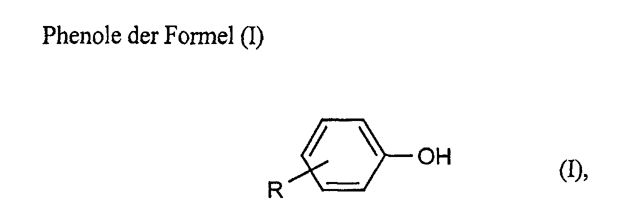 worin
- R: Wasserstoff, tert.-Butyl, Halogene oder ein verzweigter oder unverzweigter C₈-und/oder C₉-Alkylrest ist,
somit also Phenol selbst, Alkylphenole wie Kresole, p-tert.-Butylphenol, p-Cumylphenol, p-n-Octylphenol, p-iso-Octylphenol, p-n-Nonylphenol und p-iso-Nonylphenol, Halogenphenole wie p-Chlorphenol, 2,4-Dichlorphenol, p-Bromphenol und 2,4,6-Tribromphenol. Bevorzugt ist Phenol.

In dem Verfahren verwendete inerte organische Lösungsmittel sind beispielsweise Dichlormethan, Toluol, die verschiedenen Dichlorethane und Chlorpropanverbindungen, Chlorbenzol und Chlortoluol, vorzugsweise wird Dichlormethan eingesetzt.

Die Reaktionsführung wird bevorzugt kontinuierlich und bevorzugt in einer Pfropfenströmung ohne große Rückvermischung durchgeführt. Dies kann somit beispielsweise in Rohrreaktoren geschehen. Die Reaktionsführung unterteilt sich in zwei aufeinanderfolgende Stufen.

In der erste Stufe wird die wässrige Phase mit der organischen Phase in einer Blende, einer Düse oder einem dynamischen Mischer vermischt. Vorzugsweise ist eine Düse zu verwenden, beispielsweise eine mit Bohrungen versehene Konfusor-Diffusor-Kombination oder ein sprungartig verengtes Rohr.

Die wässrige Phase im erfindungsgemäßen Verfahren durchströmt die Bohrungen mit Geschwindigkeiten zwischen 2 bis 15 m/s, bevorzugt zwischen 6 und 12 m/s. Der Druckverlust variiert zwischen 0,1 und 2,5 bar, bevorzugt zwischen 0,5 und 1,5 bar. Im engsten Querschnitt hat die Mischung eine Geschwindigkeit zwischen 2 und 15 m/s und einen Druckverlust zwischen 0,1 und 2 bar.

Hinter dem Mischorgan ist ein Kühler, bevorzugt mit einem Flüssigkeitsverteiler, zur Kühlung des Reaktionsgemisches auf <50°C, bevorzugt <40°C vorzusehen.

Die erfindungsgemäßen u.g. Mischbereichsoptima mit entsprechenden Ober- und Untergrenzen für die erste und die zweite Stufe der Reaktionsführung stellen einerseits hohe Phosgenausbeuten in hohen Reinheiten des Kohlensäurediarylesters sicher und verhindern andererseits hohe Konzentrationen des Zwischenproduktes Chlorameisensäurearylester im Endprodukt bei gleichzeitig kurzen Reaktionszeiten. Bei zu geringeren Mischleistungen ist die Reaktion unvollständig, d.h. es wird das Monophenol nicht komplett mit dem Phosgen umgesetzt, so dass Monophenol zum einen ins Abwasser gelangt und zum anderen das Zwischenprodukt der Chlorameisensäurearylester ins Endprodukt geschleppt wird. Bei zu hohen Mischleistungen tritt eine höhere Verseifungs- und Zurückspaltung des Phosgens und des Kohlensäurediarylesters auf, so dass Monophenol zum einen ins Abwasser gelangt und zum anderen ein deutlich erhöhter Phosgenüberschuss benötigt wird.

Bei der Reaktion in der ersten Stufe werden die Reaktionskomponenten durch Zusammenbringen der Edukte Phosgen, inertes Lösungsmittel, welches bevorzugt erst als Lösungsmittel für das Phosgen dient, Phenol, welches vorzugsweise zuvor bereits in der Alkalilauge gelöst ist, gestartet. Die Verweilzeit im kontinuierlichen Prozess der ersten Stufe liegt im Bereich von 2 Sekunden bis 300 Sekunden, bevorzugt im Bereich von 4 Sekunden bis 200 Sekunden. Vorzugsweise wird der pH-Wert der ersten Stufe durch das Verhältnis von Alkalilauge/Phenol/Phosgen so eingestellt, dass der pH-Wert im Bereich von 11,0 bis 12,0, bevorzugt 11,2 bis 11,8 liegt.

In der zweiten Stufe des kontinuierlichen Verfahrens wird die Reaktion zum Kohlensäurediarylester komplettiert. Die Durchmischung der zwei Phasen (wässrige und organische Phase) in der zweite Stufe erfolgt in regelmäßigen Abständen durch bevorzugt statische oder dynamische Dispergierorgane. Als statische Elemente kommen dabei vorzugsweise Blenden, Düsen oder verengte Rohrquerschnitte zum Einsatz. In letztere können zusätzlich Einbauten (statische Mischer) zur Stromteilung und -vermischung eingesetzt werden. Der Druckabfall pro Dispergierelement beträgt vorzugsweise zwischen 0,1 und 0,5 bar. Die Geschwindigkeit im engsten Querschnitt des Elements liegt typischerweise zwischen 2 und 10 m/s, bevorzugt zwischen 3 und 9 m/s, ganz besonders bevorzugt zwischen 4 und 7 m/s. Die Mischzeit (Verweilzeit im Dispergierorgan) liegt unter 0,5 s, bevorzugt zwischen 0,01 und 0,1 s. Die Verweilzeit zwischen zwei aufeinanderfolgenden Dispergierorganen beträgt zwischen 3 und 12 s, bevorzugt zwischen 5 und 10 s.

Die Verweilzeiten der zweiten Stufe liegen beim erfindungsgemäßen Prozess hierbei zwischen 1 Minute und 30 Minuten, bevorzugt zwischen 2 Minuten und 20 Minuten, ganz besonders bevorzugt zwischen 3 Minuten und 15 Minuten.

Geeignete dynamische Mischer sind z.B. Pumpen oder allgemein Rotor-Stator-Systeme.

In einer bevorzugten Ausführungsform wird ein Katalysator zu Beginn der zweiten Stufe zugeführt. Direkt nach oder während der Zugabe des Katalysators wird die Reaktion des Verfahrens bevorzugt gekühlt. Die Reaktionstemperatur wird durch Kühlung < 50°C, bevorzugt < 40°C, ganz besonders bevorzugt <35°C gehalten. Es kann vorteilhaft sein den Katalysator an mehreren, bevorzugt an zwei, Stellen der zweiten Stufe der Reaktion im kontinuierlichen Verfahren zuzusetzen.

Zwischen der ersten und der zweiten Stufe kann auch eine Zwischenpufferung unter Beibehaltung der gemischten Phasen durchgeführt werden.

Die erfindungsgemäß einzusetzenden Katalysatoren sind tertiäre Amine, N-Alkylpiperidine oder Oniumsalze. Unter Oniumsalzen werden in der vorliegenden Anmeldung Verbindungen wie NR4X, wobei R ein Alkyl und/oder Arylrest und/oder ein H sein kann und X ein Anion ist, verstanden. Bevorzugt werden Tributylamin, Triethylamin und N-Ethylpiperidin verwendet. Ganz besonders bevorzugt wird N-Ethylpiperidin. Die Konzentrationen der Katalysatoren betragen 0,0001 mol-% bis 0,1 mol-%, bevorzugt 0,01 mol-% bis 0,075 mol-%, bezogen auf das eingesetzte Phenol.

Der Gehalt an Chlorameisensäurearylester im resultierenden Kohlensäurediarylester beträgt nach dem erfindungsgemäßen Verfahren <2 ppm, bevorzugt <0,5 ppm.

Vorzugsweise wird in der zweiten Stufe des Verfahrens der pH-Wert durch Messung des pH-Werts (wird im kontinuierlichen Verfahren bevorzugt online nach den bekannten Verfahren gemessen) und entsprechende Einstellung des pH-Wertes durch Zugabe der Alkalilauge geregelt. Die Menge zugeführter Alkailauge wird so eingestellt, dass der pH-Wert nach der zweiten Verfahrensstufe im Bereich von 7,5 bis 10,5, bevorzugt 8 bis 10 liegt.

Im erfindungsgemäßen Verfahren wird Phosgen in Bezug auf das Phenol im Verhältnis von 1,01 bis 1,14 mol-%, bevorzugt 1,05 bis 1,10 mol-% gefahren. Das Lösungsmittel wird so zugemischt, dass der Kohlensäurediphenylester in einer 5 bis 60-%igen Lösung, bevorzugt 20 bis 45-%igen Lösung nach der Reaktion vorliegt.

Nach der Reaktion wird die organische, den Kohlensäurediarylester enthaltende Phase üblicherweise mit einer wässrigen Flüssigkeit gewaschen und nach jedem Waschvorgang von der wässrigen Phase soweit wie möglich getrennt. Die Wäsche erfolgt bevorzugt mit entsalztem Wasser. Die Kohlensäurediarylesterlösung ist nach der Wäsche und Abtrennung der Waschflüssigkeit üblicherweise trüb. Als Waschflüssigkeit werden wässrige Flüssigkeiten zur Abtrennung des Katalysators, z. B. eine verdünnte Mineralsäure wie HCl oder H₃PO₄, und zur weiteren Reinigung vollentsalztes Wasser eingesetzt. Die Konzentration von HCl oder H₃PO₄ in der Waschflüssigkeit kann beispielsweise 0,5 bis 1,0 Gew.-% betragen. Die organische Phase wird beispielhaft und vorzugsweise zweimal gewaschen.

Als Phasentrennvorrichtungen zur Abtrennung der Waschflüssigkeit von der organischen Phase können grundsätzlich bekannte Trenngefäße, Phasenseparatoren, Zentrifugen oder Coalescer oder auch Kombinationen dieser Einrichtungen verwendet werden.

Zum Erhalt des hochreinen Kohlensäurediester wird das Lösungsmittel abgedampft. Das Abdampfen kann in mehreren Verdampferstufen erfolgen. Beispielsweise erfolgt dies durch eine oder mehrere hintereinandergeschaltete Destillationskolonnen bei der das Lösungsmittel vom Kohlensäurediarylester getrennt wird.

Diese Reinigungsstufe bzw. Stufen können beispielsweise kontinuierlich so geführt werden, dass die Sumpftemperatur bei der Destillation > 150°C bis 310°C, bevorzugt > 160 bis 230°C liegt. Der zur Durchführung dieser Destillation notwendige Druck liegt zwischen 1 und 1000 mbar, bevorzugt zwischen 5 und 100 mbar.

Die erhaltenen Kohlensäurediester zeichnen sich durch eine sehr hohe Reinheit mit GC-Reinheiten (cool on column-Methode) >99,99 %, bevorzugt 99,9925 %, ganz besonders bevorzugt >99,995 % und einem extrem guten Umesterungsverhalten aus, so dass ein Polycarbonat in excellenter Qualität hergestellt werden kann.

Die Herstellung von aromatischen Oligo-/Polycarbonaten nach dem Schmelzeumesterungsverfahren ist literaturbekannt und beispielsweise in der Encyclopedia of Polymer Science, Vol. 10 (1969), Chemistry and Physics of Polycarbonates, Polymer Reviews, H. Schnell, Vol. 9, John Wiley and Sons, Inc. (1964) oder dem US-P 5 340 905 vorbeschrieben.

### Beispiele

### Beispiel 1

In einen gekühlten Rohrreaktor wird kontinuierlich ein Gemisch aus 117 kg/h vollentsalztem Wasser mit 48 kg/h 50-%iger NaOH und 54,9 kg/h Phenol mit einer Lösung von 98 kg/h Methylenchlorid und 31,2 kg/h Phosgen (8 mol-% Überschuss bezogen auf Phenol) zusammengebracht. Beide Phasen werden in einer mit Bohrungen versehenen Konfusor-Diffusor-Düse vermischt. Der Druckverlust der wässrigen Phase (Bohrungsseite) beträgt 0,8 bar bei einer Strömungsgeschwindigkeit von 9 m/s. Der Druckverlust der organischen Phase beträgt 0,1 bar. Nach einer mittleren Verweilzeit von 15 Sekunden werden zu diesem Reaktionsgemisch nun in der zweiten Stufe des Verfahrens 6,5 kg/h 50-%iger NaOH zudosiert und direkt anschließend 1,1 kg/h (0,9-%ig in Methylenchlorid) des Katalysators N-Ethylpiperidin zugefügt und das Reaktionsgemisch unmittelbar auf 30°C gekühlt. Im weiteren wird das Reaktionsgemisch durch das Durchströmen eines mit Verengungen versehen Rohres ständig gemischt. Die Geschwindigkeiten in den Verengungen betragen 4 m/s (Anfang des Rohres) und 6 m/s (Ende des Rohres), mit entsprechenden Druckverlusten von 0,15 bar und 0,30 bar pro Verbindung und einer Verweilzeit in den Verengungen von 0,025 bis 0,040 s. Die Verweilzeit zwischen den aufeinanderfolgenden Verengungen beträgt jeweils 9 s. Die Gesamtverweilzeit beträgt ca. 300 Sekunden. Danach wird die organische von der wässrigen Phase abgetrennt. Nach Waschen mit 0,6-%iger HCl und Wasser und abschliessender Phasentrennung wird nach dem Abdampfen des Methylenchlorids ein 99,996-%iges (GC-Methode, cool on column-Methode) Diphenylcarbonat erhalten. Der Gehalt an Chlorameisensäurephenylester liegt <0,5 ppm. Das DPC eignet sich sehr gut zum Einsatz in das Schmelzeumesterungsverfahren.

### Vergleichsbeispiel 1

Wie Beispiel 1, nur erfolgt die Mischung der beiden Phasen durch eine anders konstruierte Konfusor-Diffusor-Düse, so dass die Geschwindigkeit lediglich 1,5 m/s bei einem Druckabfall von 20 mbar beträgt. Nach Waschen mit 0,6-%iger HCl und Wasser und abschliessender Phasentrennung wird nach dem Abdampfen des Methylenchlorids ein 99,85-%iges (GC-Methode, cool on column-Methode) Diphenylcarbonat erhalten. Der Gehalt an Chlorameisensäurephenylester liegt bei 1500 ppm. Das DPC eignet sich nicht zum Einsatz in das Schmelzeumesterungsverfahren.

### Vergleichsbeispiel 2

Wie Beispiel 1, nur ist die Geschwindigkeiten in den Verengungen in der zweiten Stufe des Verfahrens lediglich 1 m/s (Anfang des Rohres) und 1,5 m/s (Ende des Rohres), mit entsprechenden Druckverlusten von 0,010 bar und 0,020 bar pro Verbindung und einer Verweilzeit in den Verengungen von 0,08 bis 0,10 s.

Nach Waschen mit 0,6-%iger HCl und Wasser und abschliessender Phasentrennung wird nach dem Abdampfen des Methylenchlorids ein 99,85-%iges (GC-Methode, cool on column-Methode) Diphenylcarbonat erhalten. Der Gehalt an Chlorameisensäurephenylester liegt bei 350 ppm. Das DPC eignet sich nicht zum Einsatz in das Schmelzeumesterungsverfahren.

### Vergleichsbeispiel 3

Wie Beispiel 1, nur ist die Geschwindigkeiten in den Verengungen in der zweiten Stufe des Verfahrens 8 m/s (Anfang des Rohres) und 11,5 m/s (Ende des Rohres) bei Druckverlusten von 0,6 bar und 1,2 bar pro Verbindung und entsprechenden Verweilzeiten von 0,015 bis 0,020 s in den Verengungen. Um die gleiche DPC-Ausbeute, d.h. nahezu vollständige Umsetzung des Phenols zu erzielen, muss jetzt aber mit 33,8 kg/h Phosgen (17 mol-% Überschuss bezogen auf Phenol) gefahren werden.

Nach Waschen mit 0,6-%iger HCl und Wasser und abschliessender Phasentrennung wird nach dem Abdampfen des Methylenchlorids ein 99,995-%iges (GC-Methode, cool on column-Methode) Diphenylcarbonat erhalten. Der Gehalt an Chlorameisensäurephenylester liegt <0,5 ppm.

## Patentansprüche

1. Zweistufiges Verfahren zur Herstellung von Kohlensäurediarylestern aus Phosgen und Monophenolen in einem inerten Lösungsmittel in Gegenwart von Alkali und einer Stickstoffbase in der Phasengrenzfläche, **dadurch gekennzeichnet, dass** man die Reaktionsmischung in der ersten Stufe durch ein Durchmischungssystem derart mischt, das die Reaktionsmischung geeignete Verengungen mit einer Geschwindigkeit von 3 bis 15 m/s durchströmt und dabei einem Druckabfall zwischen 0,1 und 2,5 bar ausgesetzt ist, in der zweiten Stufe dann entsprechend eine Durchströmgeschwindigkeit von 2 bis 10 m/s bei einem Druckabfall von 0,1 bis 0,5 bar angewandt wird.

2. Verfahren gemäß Anspruch 1, **dadurch gekennzeichnet, dass** die Verweilzeit der Reaktionsmischung in der ersten Stufe 2 bis 300 Sekunden beträgt.

3. Verfahren gemäß Anspruch 1, **dadurch gekennzeichnet, dass** der pH-Wert in der ersten Stufe 11-12 beträgt.

4. Verfahren gemäß Anspruch 1, **dadurch gekennzeichnet, dass** die zweite Stufe in einem Reaktor mit mehreren Misch- bzw. Dispergierelementen durchgeführt wird.

5. Verfahren gemäß Anspruch 4, **dadurch gekennzeichnet, dass** die Verweilzeit pro Misch- bzw. Dispergierelement weniger als 0,5 Sekunden beträgt.

6. Verfahren nach Anspruch 5, **dadurch gekennzeichnet, dass** die Verweilzeit zwischen zwei aufeinanderfolgenden Misch- bzw. Dispergierelementen 3 bis 12 Sekunden beträgt.

7. Verfahren nach Anspruch 1, **dadurch gekennzeichnet, dass** die Verweilzeit in der zweiten Stufe 1 bis 30 min. beträgt.

## Claims

1. Two-stage process for producing carbonic acid diaryl esters from phosgene and monophenols in an inert solvent in the presence of alkali and a nitrogen base in the phase interface, **characterised in that** the reaction mixture is mixed by a thorough mixing system in the first stage in such a way that the reaction mixture permeates suitable constrictions at a speed of 3 to 15 m/s and, in so doing, is exposed to a drop in pressure of between 0.1 and 2.5 bar, a permeation speed of 2 to 10 m/s accordingly then being applied in the second stage with a drop in pressure of 0.1 to 0.5 bar.

2. Process according to claim 1, **characterised in that** the residence time of the reaction mixture in the first stage is 2 to 300 seconds.

3. Process according to claim 1, **characterised in that** the pH in the first stage is 11 to 12.

4. Process according to claim 1, **characterised in that** the second stage is carried out in a reactor with a plurality of mixing and dispersing elements.

5. Process according to claim 4, **characterised in that** the residence time per mixing and dispersing element is less than 0.5 second.

6. Process according to claim 5, **characterised in that** the residence time between two successive mixing and dispersing elements is 3 to 12 seconds.

7. Process according to claim 1, **characterised in that** residence time in the second stage is 1 to 30 minutes.

## Revendications

1. Procédé en deux étapes de préparation d'esters diaryliques d'acide carbonique à partir de phosgène et de monophénols dans un solvant inerte en présence d'alcalins et d'une base azotée dans la surface limite des phases, **caractérisé en ce que** le mélange réactif est mélangé dans la première étape par un système de mélange intime de telle sorte que le mélange réactif traverse des étranglements adéquats à une vitesse de 3 à 15 m/s et soit exposé à cette occasion à une chute de pression entre 0,1 et 2,5 bars et que, dans la deuxième étape, une vitesse d'écoulement de 2 à 10 m/s soit appliquée de manière correspondante pour une chute de pression de 0,1 à 0,5 bar.

2. Procédé selon la revendication 1, **caractérisé en ce que** le temps de séjour du mélange réactif dans la première étape s'élève à 2 à 300 secondes.

3. Procédé selon la revendication 1, **caractérisé en ce que** le pH dans la première étape s'élève à 11-12.

4. Procédé selon la revendication 1, **caractérisé en ce que** la deuxième étape est effectuée dans un réacteur avec plusieurs éléments de mélange ou de dispersion.

5. Procédé selon la revendication 4, **caractérisé en ce que** le temps de séjour par élément de mélange ou de dispersion s'élève à moins de 0,5 seconde.

6. Procédé selon la revendication 5, **caractérisé en ce que** le temps de séjour entre deux éléments successifs de mélange ou de dispersion s'élève à 3 à 12 secondes.

7. Procédé selon la revendication 1, **caractérisé en ce que** le temps de séjour dans la deuxième étape s'élève à 1 à 30 minutes.
